(19) Europäisches Patentamt / European Patent Office / Office européen des brevets

(11) **EP 4 257 127 A1**

(12) **EUROPEAN PATENT APPLICATION**
published in accordance with Art. 153(4) EPC

(43) Date of publication:
**11.10.2023 Bulletin 2023/41**

(21) Application number: **21900691.3**

(22) Date of filing: **03.12.2021**

(51) International Patent Classification (IPC):
*A61K 31/327* (2006.01)  *A61K 9/10* (2006.01)
*A61K 47/06* (2006.01)  *A61K 47/10* (2017.01)
*A61K 47/12* (2006.01)  *A61K 47/14* (2017.01)
*A61K 47/44* (2017.01)  *A61P 17/00* (2006.01)
*A61P 17/10* (2006.01)

(52) Cooperative Patent Classification (CPC):
**A61K 9/10; A61K 31/327; A61K 47/06;
A61K 47/10; A61K 47/12; A61K 47/14;
A61K 47/44; A61P 17/00; A61P 17/10**

(86) International application number:
**PCT/JP2021/044378**

(87) International publication number:
**WO 2022/118942 (09.06.2022 Gazette 2022/23)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.12.2020  JP 2020201896**

(71) Applicant: **Maruho Co., Ltd.**
**Osaka 531-0071 (JP)**

(72) Inventors:
• **SUZUKI Satoko**
  **Osaka-shi, Osaka 531-0071 (JP)**
• **MAEDA Mayumi**
  **Osaka-shi, Osaka 531-0071 (JP)**

(74) Representative: **Pfenning, Meinig & Partner mbB**
**Patent- und Rechtsanwälte**
**Theresienhöhe 11a**
**80339 München (DE)**

(54) **EMULSION COMPOSITION FOR EXTERNAL USE**

(57)     Provided is a skin composition which can reduce side effects of skin drying caused due to benzoyl peroxide and can keep benzoyl peroxide stable.

An emulsion topical composition for skin, containing:
(A) benzoyl peroxide;
(B) 0.3 to 12.5 wt.% of at least one liquid oil having an $\alpha$ value of 0° to 8°;
(C) 0.3 to 2.5 wt.% of at least one solid oil;
(D) at least one aqueous solvent; and
(E) at least one surfactant.

EP 4 257 127 A1

## Description

TECHNICAL FIELD

The present invention relates to a topical pharmaceutical composition useful for the treatment of acne vulgaris. More specifically, the present invention relates to an emulsion topical composition (an emulsion composition for external use) for skin containing benzoyl peroxide.

BACKGROUND ART

Acne vulgaris is a skin disease that is generally called "acne" and frequently occurs on the face, and self-care by application of a topical preparation is generally performed. Benzoyl peroxide is used for the treatment of acne vulgaris in many countries, and for example, Patent Document 1 and Patent Document 2 disclose a skin composition containing benzoyl peroxide as an active ingredient.

PRIOR ART DOCUMENTS

PATENT DOCUMENTS

Patent Document 1: JP-A-H09-87140
Patent Document 2: JP-A-2013-241469

SUMMARY OF THE INVENTION

PROBLEMS TO BE SOLVED BY THE INVENTION

Drying of the skin at an application site has been reported as a side effect of a skin composition containing benzoyl peroxide, and therefore a pharmaceutical composition having less such side effects is desired. As one of methods for preventing the drying of the application site, it is conceivable to enhance the moisturizing ability by adding an oil component to the composition, but when an oil component is added to the composition containing benzoyl peroxide, it was confirmed that the stability of benzoyl peroxide tended to decrease.

Therefore, an object of the present invention is to provide a skin composition containing benzoyl peroxide, which has less side effects of skin drying due to benzoyl peroxide and can keep benzoyl peroxide stable in the composition.

MEANS FOR SOLVING THE PROBLEMS

As a result of repeated studies to solve the above problems, the present inventor has succeeded in providing a skin composition excellent in both moisture retention and stability of benzoyl peroxide by using an emulsion base containing specific amounts of a liquid oil and a solid oil, an aqueous solvent, and a surfactant as a base of a composition containing benzoyl peroxide.

The present invention relates to the following emulsion topical composition.

[1] An emulsion topical composition for skin, containing:

(A) benzoyl peroxide;
(B) 0.3 to 12.5 wt.% of at least one liquid oil having an $\alpha$ value of 0° to 8°;
(C) 0.3 to 2.5 wt.% of at least one solid oil;
(D) at least one aqueous solvent; and
(E) at least one surfactant.

[2] The emulsion topical composition according to [1], containing only benzoyl peroxide as an active ingredient.

[3] The emulsion topical composition according to [1] or [2], wherein the liquid oil (B) is selected from the group consisting of squalane, liquid paraffin, light liquid paraffin, octyldodecyl myristate, decyl oleate, and cetyl 2-ethylhexanoate.

[4] The emulsion topical composition according to any one of [1] to [3], wherein the solid oil (C) is selected from the group consisting of aliphatic alcohols having 16 or more carbon atoms, stearic acid, paraffin, and white beeswax.

[5] The emulsion topical composition according to any one of [1] to [4], wherein a content of the liquid oil (B) is 0.5 to 10 wt.%, and a content of the solid oil (C) is 0.3 to 1.4 wt.%.

[6] The emulsion topical composition according to any one of [1] to [5], wherein the aqueous solvent (D) consists of (d1) 5 to 30 wt.% of polyhydric alcohol and (d2) 50 wt.% or more of water.

[7] The emulsion topical composition according to [1], containing: 0.3 to 12.5 wt.% of squalane as the liquid oil (B);

0.3 to 2.5 wt.% of aliphatic alcohol selected from the group consisting of stearyl alcohol, cetostearyl alcohol, and cetyl alcohol as the solid oil (C); and
(d1) 5 to 30 wt.% of polyhydric alcohol and (d2) 50 wt.% or more of water as the aqueous solvent (D).

[8] The emulsion topical composition according to [1], containing: 1.5 to 5 wt.% of squalane as the liquid oil (B);

0.3 to 0.9 wt.% of aliphatic alcohol selected from the group consisting of stearyl alcohol, cetostearyl alcohol, and cetyl alcohol as the solid oil (C); and
(d1) 10 to 25 wt.% of polyhydric alcohol and (d2) 65 wt.% or more of water as the aqueous solvent (D), wherein the total content of components other than the components [A] to [E] is 5 wt.% or less.

[9] The emulsion topical composition according to [7] or [8], containing only benzoyl peroxide as an active ingredient.

EFFECT OF THE INVENTION

Since the emulsion topical composition according to the present invention is excellent in moisture retention, the skin at the application site is hardly dried, and furthermore the stability of the active ingredient, benzoyl peroxide, is excellent.

BRIEF DESCRIPTION OF THE DRAWINGS

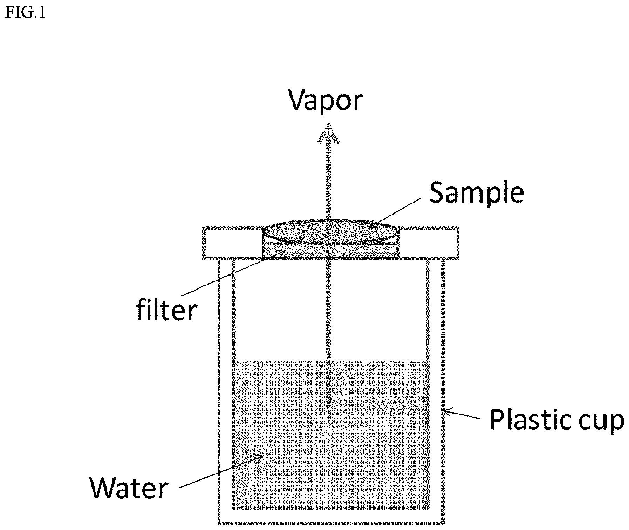

Fig. 1 is a diagram for explaining a water evaporation suppression evaluation test.

MODE FOR CARRYING OUT THE INVENTION

The emulsion topical composition of the present invention contains benzoyl peroxide (A) as an active ingredient. Benzoyl peroxide can be prepared by adding hydrogen peroxide water to a sodium hydroxide solution to obtain a sodium peroxide solution, and adding benzoyl chloride thereto. It can also be obtained from the market. The content of benzoyl peroxide is preferably 1 to 5 wt.%, more preferably 1.5 to 4 wt.%, particularly preferably 2 to 3 wt.%, and still more preferably 2.5 wt.%.

In the present specification, wt.% or content means a proportion of the weight of each component when the total weight of the emulsion topical composition is 100%.

It is preferable that the benzoyl peroxide (A) is not covered with a metal oxide layer. In addition, the emulsion topical composition of the present invention desirably contains only benzoyl peroxide as an active ingredient (ingredient for treating acne). For example, as a therapeutic agent for acne, a mixture containing a naphthoic acid compound such as adapalene and benzoyl peroxide as active ingredients is known, but the emulsion topical composition of the present invention preferably does not contain a naphthoic acid compound such as adapalene.

The emulsion topical composition of the present invention contains 0.3 to 12.5 wt.% of a liquid oil (B) having an $\alpha$ value of 0° to 8°. In the present specification, the liquid oil refers to an oily component that is liquid at normal temperature (25°C). In addition, the $\alpha$ value means an $\alpha$ value according to the organic conceptual diagram. The organic conceptual diagram was proposed by Atsushi Fujita, and the details thereof are described in "Pharmaceutical Bulletin", 1954, vol. 2, 2, pp. 163-173; "Chemistry region", 1957, vol. 11, 10, pp. 719-725; "Fragrance journal", 1981, vol. 50, pp. 79-82, and the like. That is, the source of all the organic compounds is methane ($CH_4$), and all the other compounds are regarded as derivatives of methane, and certain numerical values are set for the carbon number, the substituent, the modification part, the ring, and the like, and the scores are added to determine an organic value and an inorganic value. This value is plotted on a diagram in which the organic value is taken on an X-axis and the inorganic value is taken on a Y-axis, and the inclination is an $\alpha$ value.

When the liquid oil having an $\alpha$ value of more than 8° is used, the stability of benzoyl peroxide in the emulsion topical composition tends to be deteriorated. It is more preferable to use a liquid oil having an $\alpha$ value of 7° or less.

Preferred examples of the liquid oil (B) include liquid hydrocarbons and low-polarity fatty acid ester oils. Examples of the liquid hydrocarbons include squalane (0°), liquid paraffin (0°), light liquid paraffin (0°), light isoparaffin (0°), liquid isoparaffin (0°), squalene (1°), $\alpha$-olefin oligomer (0°), and polybutene (0°). Examples of the low-polarity fatty acid ester oils include octyldodecyl myristate (5°), oleyl oleate (5°), decyl oleate (6°), and cetyl 2-ethylhexanoate (7°). The numerical value in parentheses following each substance name represents an α value. Any of the liquid oils can be used alone or in combination.

A more preferable liquid oil is a liquid hydrocarbon oil having an α value of 0°, such as squalane, liquid paraffin, and light liquid paraffin, and squalane is particularly preferable.

The content of the liquid oil (B) is preferably 0.5 to 10 wt.%, more preferably 1 to 8 wt.%, particularly preferably 1.5 to 5 wt.%, and still more preferably 2 to 4 wt.%. Too little liquid oil reduces the moisturizing ability, whereas too much liquid oil reduces the stability of benzoyl peroxide.

The emulsion topical composition of the present invention contains 0.3 to 2.5 wt.% of a solid oil (C). In the present specification, the solid oil refers to an oily component that is semisolid or solid at normal temperature (25°C).

Preferred examples of the solid oil (C) include aliphatic alcohols having 16 or more carbon atoms, fatty acids having 18 or more carbon atoms, hydrocarbons, and waxes. Examples of the aliphatic alcohol having 16 or more carbon atoms include saturated aliphatic alcohols (particularly linear ones) having 16 to 22 carbon atoms (the number of carbon atoms is more preferably 16 to 20, and particularly preferably 16 to 18) and having one hydroxyl group only at the terminal carbon, and particularly preferable examples include stearyl alcohol (18 carbon atoms), cetanol (also referred to as cetyl alcohol: 16 carbon atoms), and cetostearyl alcohol (a mixture of approximately equal amounts of stearyl alcohol and cetanol). Any of the solid oils can be used alone or in combination. Examples of the fatty acid having 18 or more carbon atoms include stearic acid (18 carbon atoms) and behenic acid (22 carbon atoms). Examples of the hydrocarbon include paraffin and microcrystalline wax. Examples of waxes include white beeswax and lanolin.

The content of the solid oil (C) is more preferably 0.3 to 2 wt.%, particularly preferably 0.3 to 1.4 wt.%, and still more preferably 0.3 to 0.9 wt.%. As the amount of the solid oil (C) increases, the moisture retention of the emulsion topical composition increases, but on the other hand, the stability of benzoyl peroxide decreases.

Although it is possible to achieve the desired moisture retention using only a large amount of the liquid oil (B) and the solid oil (C), in that case, the desired stability cannot be achieved because benzoyl peroxide becomes unstable. Therefore, the emulsion topical composition of the present invention is characterized by containing a specific amount of liquid oil and a specific amount of solid oil in combination. The total amount of the liquid oil and the solid oil is preferably at least 0.6 wt.%, more preferably 1.0 wt.% or more, particularly preferably 1.5 wt.% or more, still more preferably 2.4 wt.% or more. The total amount of the liquid oil and the solid oil is preferably 12 wt.% or less, more preferably 10 wt.% or less, particularly preferably 8 wt.% or less, still more preferably 6 wt.% or less.

The emulsion topical composition of the present invention contains an aqueous solvent (D). Preferred examples of the aqueous solvent include water (preferably purified water) and polyhydric alcohol. Examples of the polyhydric alcohol include polyhydric alcohol having 3 to 6 carbon atoms. The polyhydric alcohol selected from glycerin, propylene glycol, and 1,3-butylene glycol is more preferable, and glycerin and 1,3-butylene glycol are particularly preferable. Any of the aqueous solvents can be used alone or in combination.

The content of the aqueous solvent (D) is preferably 60 wt.% or more, more preferably 70 wt.% or more, particularly preferably 80 wt.% or more, and still more preferably 85 wt.% or more. The upper limit of the aqueous solvent (D) is preferably 97 wt.% or less, and more preferably 95 wt.% or less.

In addition, the emulsion topical composition preferably contains, as the aqueous solvent (D), (d1) polyhydric alcohol and (d2) water (preferably purified water), more preferably contains 5 to 30 wt.% of polyhydric alcohol and 50 wt.% or more of water, particularly preferably contains 10 to 25 wt.% of polyhydric alcohol and 65 wt.% or more of water, and still more preferably contains 10 to 20 wt.% of polyhydric alcohol and 70 wt.% or more of water. The upper limit of the water (d2) is preferably 95 wt.% or less, more preferably 90 wt.% or less, particularly preferably 85 wt.% or less, and still more preferably 80 wt.% or less. The wt.% of the above (d1) and (d2) means not the wt.% based on the total amount of the aqueous solvent (D) but the wt.% when the total amount of the emulsion topical composition is 100 wt.%.

As the aqueous solvent (D), lower monohydric alcohol (monohydric alcohol having 1 to 3 carbon atoms such as ethanol and isopropanol) may be used, but since the lower monohydric alcohol has skin irritation, it is preferable that the emulsion topical composition of the present invention substantially does not contain the lower monohydric alcohol. In the present specification, "substantially does not contain" a substance means that the substance is not intentionally added at the time of preparing the emulsion topical composition, and therefore the content of the substance in the emulsion topical composition is usually less than 1 wt.%, more preferably less than 0.5 wt.%, and particularly preferably 0 wt.%.

The emulsion topical composition of the present invention contains a surfactant (E). Examples of the surfactant (emulsifier) include a nonionic surfactant, a cationic surfactant, an anionic surfactant, and an amphoteric surfactant.

Examples of nonionic surfactants include polyethylene glycol fatty acid esters such as polyethylene glycol monostearate, ethylene glycol monostearate, polyethylene glycol monolaurate, ethylene glycol monostearate, and polyethylene glycol monooleate; polyoxyethylene alkyl ethers such as polyoxyethylene behenyl ether, polyoxyethylene oleyl ether, polyoxyethylene octyldodecyl ether, polyoxyethylene cetyl ether, polyoxyethylene stearyl ether, and polyoxyethylene lauryl ether; polyoxyethylene alkylphenol ether; polyoxyethylene hydrogenated castor oil; glycerin fatty acid esters such as glyceryl monostearate, lipophilic glyceryl monostearate, self-emulsifying glyceryl monostearate, glyceryl myristate, glyceryl monoisostearate, and lipophilic glyceryl monooleate; polyglycerin fatty acid esters such as polyglyceryl monostearate, polyglyceryl monooleate, polyglyceryl monolaurate, polyglyceryl monomyristate, and decaglyceryl pentastearate; sorbitan fatty acid esters such as sorbitan monopalmitate, sorbitan monostearate, sorbitan monooleate, sorbitan coconut oil fatty acid, sorbitan tristearate, and sorbitan trioleate; polyoxyethylene sorbitan fatty acid esters such as polysorbate 60, polysorbate 65, polysorbate 80, and polysorbate 85; polyoxyethylene polyoxypropylene alkyl ethers such as polyoxyethylene polyoxypropylene cetyl ether (20E.O.) (4P.O.) and polyoxyethylene polyoxypropylene cetyl ether (20E.O.) (8P.O.); and plyoxyethylene beeswax derivatives such as polyoxyethylene sorbitol beeswax.

Examples of cationic surfactants include cetyltrimethylammonium chloride, lauryldimethylbenzylammonium chloride, tetrabutylammonium chloride, and dioctadecyldimethylammonium chloride.

Examples of the anionic surfactant include sodium alkylbenzene sulfonate, sodium dodecyl sulfate, sodium cocoalcohol ethoxy sulfate, sodium $\alpha$-olefin sulfonate, and emulsified cetostearyl alcohol.

Examples of the amphoteric surfactant include N-alkyl-N,N-dimethylammonium betaine and an imidazoline-type amphoteric surfactant.

Any of the surfactants (E) can be used alone or in combination. Preferred surfactants are nonionic surfactants, more preferably polyethylene glycol fatty acid esters. As described above, the emulsion topical composition of the present invention may contain polyoxyethylene alkyl ether (nonionic surfactant), but it is preferable that the emulsion topical composition substantially does not contain polyoxyethylene alakyl ether (ether composed of polyoxyethylene and arachidyl alcohol which is aliphatic alcohol having 20 carbon atoms).

The content of the surfactant (E) is preferably 0.05 to 2 wt.%, more preferably 0.1 to 1 wt.%, and particularly preferably 0.2 to 0.5 wt.%.

The emulsion topical composition of the present invention may contain one or more components other than the above (A) to (E), but the total amount of these components is preferably 5 wt.% or less, more preferably 3 wt.% or less, and particularly preferably 2 wt.% or less. In particular, it is preferable that the emulsion topical composition of the present invention substantially does not contain a substance selected from the group consisting of a polyurethane polymer type compound or a derivative thereof, a naphthoic acid derivative such as adapalene, polyoxyethylene alakyl ether, non-irritating benzoic acid ester (such as $C_{12}$ to $C_{15}$ alkyl benzoate), and a film forming agent (such as polyvinylpyrrolidone).

The pH value of the emulsion topical composition of the present invention is preferably 3 to 7. When the pH is less than 3, there is irritation to the skin, and when the pH is more than 7, the stability of benzoyl peroxide is deteriorated. A more preferred pH value is 4 to 6 and a particularly preferred pH value is 4 to 5. Examples of the pH adjuster include diisopropanolamine, triisopropanolamine, triethanolamine, potassium hydroxide, sodium hydroxide, sodium citrate, phosphoric acid, tartaric acid, dl-malic acid, and glacial acetic acid. Any of the pH adjusters can be used alone or in combination.

The emulsion topical composition of the present invention may contain a thickener. Examples of the thickener include a carboxyvinyl polymer and carboxymethylcellulose, and a carboxyvinyl polymer is particularly preferable. The content of the thickener is preferably 0.2 to 1.5 wt.%, more preferably 0.3 to 1.0 wt.%, and particularly preferably 0.4 to 0.8 wt.%.

The emulsion topical composition of the present invention can contain other additives commonly used in topical preparations for skin. Examples of such additives include antioxidants (ascorbic acid, dibutylhydroxytoluene, $\alpha$-tocopherol, tocopherol acetate, and the like), stabilizers (edetate sodium hydrate, 1-menthol, and the like), preservatives (paraoxybenzoic acid ester, phenoxyethanol, and the like), and colorants (titanium oxide, and the like).

Examples of the dosage form of the emulsion topical composition of the present invention include emulsion lotion, water-in-oil (W/O type) cream, oil-in-water (O/W type) cream, topical aerosol, and pump spray. In particular, emulsion lotion (emulsion type liquid composition) is preferred. The emulsion lotion is an oil-in-water (O/W) liquid composition obtained by emulsifying an oily component (a component that does not mix with water) and an aqueous component (water and a component that mixes with water) with a surfactant. Since the emulsion lotion contains an oil component, the emulsion lotion has higher moisture retention than an aqueous gel containing no oily component. On the other hand, since it is liquid and has low viscosity, it has a fresh feel, and it is less likely to cause stickiness, shiny, and the like at the application site, so that it is suitable for application to the face, which is a common site of acne vulgaris.

As a preferred example of the emulsion topical composition of the present invention, there is provided an emulsion topical composition (particularly emulsion lotion), containing:

(A) benzoyl peroxide;

(B) 0.3 to 12.5 wt.% (preferably 0.5 to 10 wt.%, more preferably 1 to 8 wt.%, particularly preferably 1.5 to 5 wt.%, and still more preferably 2 to 4 wt.%) of at least one liquid oil selected from the group consisting of squalane, liquid paraffin, light liquid paraffin, octyldodecyl myristate, decyl oleate, and cetyl 2-ethylhexanoate;

(C) 0.3 to 2.5 wt.% (preferably 0.3 to 2.0 wt.%, more preferably 0.3 to 1.4 wt.%, particularly preferably 0.3 to 0.9 wt.%, and still more preferably 0.4 to 0.9 wt.%) of at least one solid oil selected from the group consisting of aliphatic alcohols having 16 or more carbon atoms, stearic acid, paraffin, and white beeswax;
(D) an aqueous solvent composed of (d1) 5 to 30 wt.% (preferably 10 to 25 wt.%, more preferably 10 to 20 wt.%) of polyhydric alcohol and (d2) 50 wt.% or more (preferably 65 wt.% or more, more preferably 70 wt.% or more) of water; and
(E) at least one surfactant. In particular, an emulsion topical composition having a total content of components other than the components (A) to (E) of 5 wt.% or less, 3 wt.% or less, or 2 wt.% or less is preferable.

As a particularly preferred example of the emulsion topical composition of the present invention, there is provided an emulsion topical composition (particularly emulsion lotion), containing:

(A) benzoyl peroxide;
(B) 0.3 to 12.5 wt.% (preferably 0.5 to 10 wt.%, more preferably 1 to 8 wt.%, particularly preferably 1.5 to 5 wt.%, and still more preferably 2 to 4 wt.%) of squalane;
(C) 0.3 to 2.5 wt.% (preferably 0.3 to 2.0 wt.%, more preferably 0.3 to 1.4 wt.%, particularly preferably 0.3 to 0.9 wt.%, and still more preferably 0.4 to 0.9 wt.%) of at least one solid oil selected from the group consisting of stearyl alcohol, cetostearyl alcohol and cetyl alcohol;
(D) an aqueous solvent composed of (d1) 5 to 30 wt.% (preferably 10 to 25 wt.%, more preferably 10 to 20 wt.%) of polyhydric alcohol selected from the group consisting of glycerin, 1,3-butylene glycol, and propylene glycol and (d2) 50 wt.% or more (preferably 65 wt.% or more, more preferably 70 wt.% or more) of water; and
(E) at least one surfactant. In particular, an emulsion topical composition having a total content of components other than the components (A) to (E) of 5 wt.% or less, 3 wt.% or less, or 2 wt.% or less is preferable.

The emulsion topical composition of the present invention can be used for the treatment of acne. For example, but not limited to, the emulsion topical composition can be used in the treatment of acne vulgaris, comedone acne or polymorph acne, acne rosacea, nodular cystic acne, acne conglobata, senile or secondary acne (for example, solar acne), drug-induced or occupational acne. The application amount and application frequency may be appropriately adjusted according to the degree of symptoms, the concentration of benzoyl peroxide in the emulsion topical composition, and the like. For example, in the case of use on the face, once or twice a day, after face washing, an amount to cover the affected part is applied to the skin in which acne vulgaris occurs.
In the preceding paragraph, preferred compound names of essential components and optional components used in the composition of the present invention have been described, but the composition of the present invention also includes a composition obtained by optionally combining these components and a composition obtained by optionally combining the contents of the respective components. In addition, numerical ranges related to each component, pH, and the like can be optionally combined, and in a case where a plurality of numerical ranges is described, an upper limit value or a lower limit value of each numerical range can also be optionally combined.
Hereinafter, the present invention will be described in more detail with reference to Examples, but the present invention is not limited to Examples. % for each component in Examples means wt.%. As a surfactant, a nonionic surfactant was used.

[Example 1]

Formulations (emulsion lotions) having the compositions shown in Tables 1 and 2 were prepared. Thereafter, in order to evaluate the moisture retention, the water evaporation suppression rate was measured for each formulation by the following method. When a sensory test was conducted by a person as a panelist, the formulation having a water evaporation suppression rate of 25% or more was evaluated as maintaining a moist feeling after application of the formulation (high moisture retention). Therefore, the formulation having a water evaporation suppression rate of 25% or more was evaluated as having a moisturizing ability "good", and the formulation having a water evaporation suppression rate of less than 25% was evaluated as having a moisturizing ability "defective".

<Measurement of water evaporation suppressing ability>

A circle having a diameter of 4 cm was opened at the center of a plastic cup lid, and a carrier membrane was attached to this portion (see Fig. 1). This lid was placed on a cup containing 50 g of water, and then 100 μL (about 0.1 g) of the formulation was applied to the carrier membrane. As a control for comparison, a carrier membrane without application of a formulation was used. As the carrier membrane, a membrane filter (pore size: 10 μm, diameter: 47 mm) made of hydrophobic polytetrafluoroethylene manufactured by Millipore was used.
After storage in an environment of 30°C/40% RH for 4 hours, the amount of water evaporated from the cup of the formulation application group through the carrier membrane was defined as WLx (g/h), the amount of water evaporated from the cup of the formulation non-application group through the carrier membrane was defined as $WL_0$ (g/h), and the water evaporation suppression rate (%), which is a value showing an occlusive effect, was calculated using the following formula.

$$\text{Water evaporation suppression rate (\%)} = \frac{(WL_0 - WL_X)}{WL_0} \times 100$$

[Table 1]

| Formulation No. | | Formulation 1 | Formulation 2 | Formulation 3 | Formulation 4 | Formulation 5 | Formulation 6 | Formulation 7 | Formulation 8 | Formulation 8A | Formulation 8B | Formulation 8C |
|---|---|---|---|---|---|---|---|---|---|---|---|---|
| Active Ingredient | Benzoyl peroxide | 2.5 | 2.5 | 2.5 | 2.5 | 2.5 | 2.5 | 2.5 | 2.5 | 2.5 | 2.5 | 2.5 |
| Polyhydric alcohol | Glycerin | 5 | 5 | 5 | 5 | 5 | 10 | 20 | | | | |
| | 1,3-butylene glycol | 1 | 1 | 1 | 1 | 1 | 1 | 1 | 1 | 1 | 1 | 1 |
| Solid oil | Cetostearyl alcohol (C16&C18) | | 0.25 | 0.5 | 1.0 | 1.8 | | | 0.5 | 0.8 | 1.3 | 1.5 |
| Liquid oil ($\alpha$ value) | Squalane (0°) | 3 | 3 | 3 | 3 | 3 | 3 | 3 | 3 | 10 | 8 | 12.5 |
| Surfactant | | 0.3 | 0.3 | 0.3 | 0.3 | 0.3 | 0.3 | 0.3 | 0.3 | 0.3 | 0.3 | 0.3 |
| Water | Purified water | q. s. | q. s. | q. s. | q.s. | q.s. | q. s. | q.s. | q.s. | q. s. | q. s. | q. s. |
| Moisturizing ability | Water evaporation suppression rate (%) | 9.33 | 21.02 | 30.35 | 35.65 | 35.10 | 21.09 | 22.50 | 37.78 | 32.05 | 33.28 | 42.64 |
| | Evaluation | Defective | Defective | Good | Good | Good | Defective | Defective | Good | Good | Good | Good |

* Additives (thickener, stabilizer, pH adjuster, and the like) are appropriately added (the total amount of additives is 3 wt.% or less).

The numerical value of each component shown in Table 1 is wt.%, and the blank means 0%. The "q.s." of purified water means an amount of purified water that achieves a total formulation amount of 100 wt.% (the same applies to other tables).

As shown in Table 1, Formulation 1 containing 0% of a solid oil or Formulation 2 containing 0.25% of a solid oil had a low water evaporation suppression rate, and did not have sufficient moisturizing ability. On the other hand, Formulations 3 to 5 having a solid oil content of 0.5% to 1.8% had good moisture retention. Formulations 6 and 7 (containing no solid oil) containing a larger amount of polyhydric alcohol than Formulation 1 exhibited a higher water evaporation suppression rate than Formulation 1, but the water evaporation suppression rate did not reach 25%. On the other hand, when 0.5% of the solid oil was contained, Formulation 8 having a smaller amount of polyhydric alcohol than Formulation 3 exhibited good moisturizing ability. From these results, it was found that a formulation containing a predetermined amount of a solid oil exhibits a high moisturizing ability, but a formulation not containing a solid oil cannot achieve a desired moisturizing ability even if a large amount of glycerin generally known as a moisturizing agent is contained.

The formulations (Formulations 8A to 8C) containing 0.5% or more of the solid oil and increased in the solid oil and the liquid oil also exhibited a water evaporation suppression rate of 30% or more, and had a high moisturizing ability.

The results of changing the type of solid oil are shown in Table 2.

[Table 2]

| Formulation No. | | Formulation 9 | Formulation 10 | Formulation 11 | Formulation 12 | Formulation 13 | Formulation 14 |
|---|---|---|---|---|---|---|---|
| Active Ingredient | Benzoyl peroxide | 2.5 | 2.5 | 2.5 | 2.5 | 2.5 | 2.5 |
| Polyhydric alcohol | Glycerin | 5 | 5 | 5 | 5 | 5 | 5 |
| | 1,3-butylene glycol | 1 | 1 | 1 | 1 | 1 | 1 |
| Solid oil | Stearyl alcohol (C18) | 1.8 | | | | | |
| | Cetostearyl alcohol (C16&C18) | | 1.8 | | | | |
| | Cetyl alcohol (C16) | | | 1.8 | | | |
| | Stearic acid (C18) | | | | 1.8 | | |
| | Paraffin | | | | | 1.8 | |
| | White beeswax | | | | | | 1.8 |
| Liquid oil ($\alpha$ value) | Squalane (0°) | 3 | 3 | 3 | 3 | 3 | 3 |
| Surfactant | | 0.3 | 0.3 | 0.3 | 0.3 | 0.3 | 0.3 |
| Water | Purified water | q.s. | q.s. | q. s. | q.s. | q.s. | q.s. |
| Moisturizing ability | Water evaporation suppression rate (%) | 35.80 | 35.10 | 41.38 | 33.57 | 61.99 | 49.50 |
| | Evaluation | Good | Good | Good | Good | Good | Good |
| * Additives (thickener, stabilizer, pH adjuster, and the like) are appropriately added (the total amount of additives is 3 wt.% or less). | | | | | | | |

As shown in Table 2, the formulations prepared using aliphatic alcohols having 16 to 18 carbon atoms, stearic acid, paraffin, or white beeswax as solid oils all showed good water evaporation suppression rate.

[Example 2]

Each formulation (emulsion lotion) having the composition shown in Tables 3 to 5 was prepared and tested for the stability of an active ingredient, benzoyl peroxide. After storage at 50°C for 2 weeks, a formulation in which the amount of benzoic acid as a decomposition product of benzoyl peroxide was 10% or less was evaluated as stability "good", and a formulation in which the amount of benzoic acid was more than 10% was evaluated as stability "defective". The stability test method is as follows.

<Method of stability test>

An amount of the formulation corresponding to about 50 mg of benzoyl peroxide was weighed, and tetrahydrofuran and acetonitrile were added thereto for dispersion extraction. Tetrahydrofuran was further added to the extract, and the mixture was filtered to obtain a sample solution.
Separately, about 0.1 g of benzoic acid was weighed, and acetonitrile was added to obtain a standard solution. The sample solution and the standard solution were tested by liquid chromatography, and the amount of benzoic acid contained in the formulation was measured.

Test conditions

Detector: Ultraviolet absorption photometer (measurement wavelength: 235 nm)
Column: A stainless steel tube having an inner diameter of 4.6 mm and a length of 15 cm, packed with octadecylsilylated silica gel for liquid chromatography of 5 $\mu$m.
Mobile phase A: Water/acetic acid (100) mixed solution (1000: 1)
Mobile phase B: Acetonitrile/acetic acid (100) mixed solution (1000: 1)

The concentration gradient is controlled from the mobile phase A-rich to the mobile phase B-rich by changing a mixing ratio of the mobile phase A and the mobile phase B.

[Table 3]

| | Formulation No. | Formulation 15 | Formulation 16 | Formulation 17 | Formulation 18 | Formulation 19 | Formulation 20 | Formulation 21 | Formulation 22 | Formulation 23 |
|---|---|---|---|---|---|---|---|---|---|---|
| Active Ingredient | Benzoyl peroxide | 2.5 | 2.5 | 2.5 | 2.5 | 2.5 | 2.5 | 2.5 | 2.5 | 2.5 |
| Polyhydric alcohol | 1,3-butylene glycol | 1 | 1 | 1 | 1 | 1 | 1 | 1 | 1 | 1 |
| Solid oil | Cetostearyl alcohol (C16&C18) | 0.5 | 0.5 | 0.5 | 0.5 | 0.5 | 0.5 | 0.5 | 0.5 | 0.5 |
| Liquid oil ($\alpha$ value) | Squalane (0°) | 3 | | | | | | | | |
| | Liquid paraffin (0°) | | 3 | | | | | | | |
| | Light liquid paraffin (0°) | | | 3 | | | | | | |
| | Octyldodecyl myristate (5°) | | | | 3 | | | | | |
| | Decyl oleate (6°) | | | | | 3 | | | | |
| | Cetyl 2-ethylhexanoate (7°) | | | | | | 3 | | | |
| | Isopropyl palmitate (9°) | | | | | | | 3 | | |
| | Medium chain fatty acid triglyceride (18°) | | | | | | | | 3 | |
| | Diisopropyl sebacate (22°) | | | | | | | | | 3 |
| | Surfactant | 0.3 | 0.3 | 0.3 | 0.3 | 0.3 | 0.3 | 0.3 | 0.3 | 0.3 |
| Water | Purified water | q.s. | q.s. | q. s. | q. s. | q. s. | q. s. | q. s. | q. s. | q. s. |
| Stability test (50°C) | Benzoic acid amount (%) — At start | 0.31 | 0.26 | 0.25 | 0.27 | 0.27 | 0.25 | 0.27 | 0.26 | 0.26 |
| | Benzoic acid amount (%) — 1W | 2.65 | 2.21 | 2.45 | 3.58 | 2.79 | 3.30 | 5.46 | 7.74 | 11.11 |
| | Benzoic acid amount (%) — 2W | 6.42 | 5.03 | 5.54 | 7.76 | 5.82 | 7.51 | 10.96 | 14.36 | 20.73 |
| | Evaluation | Good | Good | Good | Good | Good | Good | Defective | Defective | Defective |
| * Additives (thickener, stabilizer, pH adjuster, and the like) are appropriately added (the total amount of additives is 3 wt.% or less). | | | | | | | | | | |

As shown in Table 3, when a liquid oil having an $\alpha$ value of 0° to 7° was used (Formulations 15 to 20), the desired active ingredient stability could be achieved. On the other hand, when a liquid oil having an $\alpha$ value of 9° to 22° was used (Formulations 21 to 23), the desired stability could not be achieved.

[Table 4]

| | | Formulation 24 | Formulation 25 | Formulation 26 | Formulation 27 | Formulation 28 | Formulation 29 | Formulation 30 | Formulation 31 | Formulation 32 |
|---|---|---|---|---|---|---|---|---|---|---|
| Active Ingredient | Benzoyl peroxide | 5 | 5 | 10 | 10 | 2.5 | 2.5 | 2.5 | 2.5 | 2.5 |
| Polyhydric alcohol | Glycerin | | | | | | | | | 15 |
| | 1,3-butylene glycol | 1 | 1 | 1 | 1 | 1 | 1 | 1 | 1 | 1 |
| Solid oil | Cetostearyl alcohol (C16&C18) | 0.5 | 0.5 | 0.5 | 0.5 | 0.5 | 0.5 | 0.5 | 0.5 | 1 |
| Liquid oil ($\alpha$ value) | Squalane (0°) | 3 | | 3 | | 3 | 3 | 3 | 3 | 1.5 |
| | Octyldodecyl myristate (5°) | | 3 | | 3 | | | | | |
| Surfactant | | 0.3 | 0.3 | 0.3 | 0.3 | 0.3 | 0.3 | 0.3 | 0.3 | 0.15 |
| Water | Purified water | q. s. | q.s. | q. s. | q. s. | q.s. | q. s. | q.s. | q. s. | q. s. |
| pH | | | | | | 3.25 | 5.05 | 5.86 | 6.98 | |
| Stability test (50°C) | Benzoic acid amount (%) At start | 0.25 | 0.23 | 0.23 | 0.22 | 0.24 | 0.23 | 0.23 | 0.26 | 0.19 |
| | 1W | 1.36 | 2.14 | 0.84 | 1.22 | 3.03 | 3.30 | 3.32 | 4.76 | 2.53 |
| | 2W | 2.74 | 4.27 | 1.62 | 2.30 | 6.61 | 7.05 | 7.47 | 9.08 | 5.98 |
| Evaluation | | Good | Good | Good | Good | Good | Good | Good | Good | Good |
| * Additives (thickener, stabilizer, pH adjuster, and the like) are appropriately added (the total amount of additives is 3 wt.% or less). | | | | | | | | | | |

As shown in Table 4, the desired stability could also be achieved when the amount of benzoyl peroxide was increased to 5% or 10% (Formulations 24 to 27), when the pH value was changed (Formulations 28 to 31), or when the amount of polyhydric alcohol was increased and the amount of surfactant was changed (Formulation 32). As the pH value increased, the stability tended to decrease (the amount of benzoic acid as a decomposition product of the active ingredient increase), but the desired stability could be achieved up to about pH 7.

[Table 5]

| Formulation No. | | Formulation 33 | Formulation 34 | Formulation 35 | Formulation 36 | Formulation 37 | Formulation 38 | Formulation 39 | Formulation 40 | Formulation 40A | Formulation 40B |
|---|---|---|---|---|---|---|---|---|---|---|---|
| Active Ingredient | Benzoyl peroxide | 2.5 | 2.5 | 2.5 | 2.5 | 2.5 | 2.5 | 2.5 | 2.5 | 2.5 | 2.5 |
| Polyhydric alcohol | 1,3-butylene glycol | 1 | 1 | 1 | 1 | 1 | 1 | 1 | 1 | 1 | 1 |
| Solid oil | Cetostearyl alcohol (C16&C18) | 0.5 | 1 | 2 | 3 | 1 | 0.5 | 1 | 0.5 | 0.4 | 0.4 |
| Liquid oil ($\alpha$ value) | Squalane (0°) | 0.5 | 0.5 | 0.5 | 0.5 | 3 | 4.5 | 4.5 | 6 | 0.4 | 1 |
| Surfactant | | 0.3 | 0.3 | 0.3 | 0.3 | 0.3 | 0.3 | 0.3 | 0.3 | 0.3 | 0.3 |
| Water | Purified water | q.s. | q.s. | q.s. | q.s. | q.s. | q.s. | q.s. | q.s. | q.s. | q. s. |
| Stability test (50°C) | Benzoic acid amount (%) — At start | 0.24 | 0.24 | 0.23 | 0.24 | 0.22 | 0.22 | 0.22 | 0.23 | 0.19 | 0.19 |
| | 1W | 2.01 | 2.10 | 2.92 | 3.18 | 2.57 | 2.51 | 2.88 | 2.67 | 1.96 | 2.02 |
| | 2W | 4.06 | 4.88 | 8.53 | 10.92 | 5.83 | 5.55 | 6.86 | 6.70 | 4.17 | 4.42 |
| | Evaluation | Good | Good | Good | Defective | Good | Good | Good | Good | Good | Good |
| * Additives (thickener, stabilizer, pH adjuster, and the like) are appropriately added (the total amount of additives is 3 wt.% or less). | | | | | | | | | | | |

EP 4 257 127 A1

16

As shown in Table 5, it was observed that the stability of benzoyl peroxide tended to decrease as the amount of solid oil increased (Formulations 33 to 36), and when the amount of solid oil was 3% (Formulation 36), the desired stability could not be achieved. On the other hand, formulations with 1% or less solid oil (Formulations 37 to 40) showed the desired stability even when liquid oil was increased to 6%. In addition, the formulations with a small amount of solid oil (Formulations 40A and 40B) had a benzoic acid amount of less than 5%, and exhibited very high stability.

[Example 3]

Formulations (emulsion lotions) having different amounts of solid oil and liquid oil were prepared, and a moisturizing ability test and a stability test were performed. The results are shown in Table 6. Each test method is as described in Example 1 and Example 2.

[Table 6]

| Formulation No. | | Formulation n41 | Formulation n42 | Formulation 43 | Formulation 44 | Formulation n45 | Formulation n46 | Formulation n47 |
|---|---|---|---|---|---|---|---|---|
| Active Ingredient | Benzoyl peroxide | 2.5 | 2.5 | 2.5 | 2.5 | 2.5 | 2.5 | 2.5 |
| Polyhydric alcohol | 1,3-butylene glycol | 1 | 1 | 1 | 1 | 1 | 1 | 1 |
| Solid oil | Cetostearyl alcohol (C16&C18) | 0.5 | 0.4 | 0.4 | 0.4 | 0.4 | 1.5 | 0.5 |
| Liquid oil ($\alpha$ value) | Squalane (0°) | 0.5 | 2 | 4 | 8 | 10 | 5 | 2.5 |
| Surfactant | | 0.3 | 0.3 | 0.3 | 0.3 | 0.3 | 0.3 | 0.3 |
| Water | Purified water | q.s. | q.s. | q. s. | q. s. | q. s. | q.s. | q.s. |
| Moisturizing ability | Water evaporation suppression rate (%) | 27.96 | 35.73 | 36.48 | 43.53 | 48.79 | 44.54 | 38.23 |
| | Evaluation | Good | Good | Good | Good | Good | Good | Good |
| Stability test (50°C) | Benzoic acid amount (%) At start | 0.24 | 0.19 | 0.18 | 0.19 | 0.19 | 0.19 | 0.19 |
| | Benzoic acid amount (%) 1W | 2.01 | 2.05 | 2.41 | 2.65 | 2.79 | 3.28 | 2.12 |
| | Benzoic acid amount (%) 2W | 4.06 | 4.49 | 5.42 | 6.67 | 7.77 | 8.54 | 4.76 |
| | Evaluation | Good | Good | Good | Good | Good | Good | Good |
| * Additives (thickener, stabilizer, pH adjuster, and the like) are appropriately added (the total amount of additives is 3 wt.% or less). | | | | | | | | |

As shown in Table 6, as the amount of the liquid oil was increased from 0.5% to 10% (Formulations 41 to 45), the water evaporation suppression rate tended to increase (the moisturizing ability was improved). On the other hand, the stability tended to gradually decrease as the amount of liquid oil increased, but the desired stability could be achieved even when the amount of liquid oil was increased to 10%. Also, a formulation of 1.5% solid oil, 5% liquid oil (Formulation 46), and a formulation of 0.5% solid oil, 2.5% liquid oil (Formulation 47) could achieve both the desired moisturizing ability and stability.

[Example 4]

Formulations (emulsion lotions) with different types of solid oils were prepared and subjected to a stability test. The results are shown in Table 7. The test method is as described in Example 2.

[Table 7]

| Formulation No. | | | Formulation 48 | Formulation 49 | Formulation 50 |
|---|---|---|---|---|---|
| Active Ingredient | Benzoyl peroxide | | 2.5 | 2.5 | 2.5 |
| Polyhydric alcohol | Glycerin | | 5 | 5 | 5 |
| | 1,3-butylene glycol | | 1 | 1 | 1 |
| Solid oil | Stearic acid (C18) | | 1.8 | | |
| | Paraffin | | | 1.8 | |
| | White beeswax | | | | 1.8 |
| Liquid oil ($\alpha$ value) | Squalane (0°) | | 3 | 3 | 3 |
| Surfactant | | | 0.3 | 0.3 | 0.3 |
| Water | Purified water | | q. s. | q.s. | q. s. |
| Stability test (50°C) | Benzoic acid amount (%) | At start | 0.28 | 0.27 | 0.28 |
| | | 1W | 4.21 | 2.49 | 2.19 |
| | | 2W | 8.89 | 4.93 | 5.23 |
| | Evaluation | | Good | Good | Good |
| * Additives (thickener, stabilizer, pH adjuster, and the like) are appropriately added (the total amount of additives is 3 wt.% or less). | | | | | |

As shown in Table 7, when fatty acid (Formulation 48), hydrocarbon (Formulation 49), and wax (Formulation 50) were used as the solid oil, the desired stability could be achieved.

[Example 5]

Formulations (emulsion lotions) with different types of liquid oils were prepared and subjected to a moisturizing ability test. The results are shown in Table 8. The test method is as described in Example 1.

[Table 8]

| Formulation No. | | Formulation 51 | Formulation 52 | Formulation 53 | Formulation 54 |
|---|---|---|---|---|---|
| Active Ingredient | Benzoyl peroxide | 2.5 | 2.5 | 2.5 | 2.5 |
| Polyhydric alcohol | 1,3-butylene glycol | 1 | 1 | 1 | 1 |
| Solid oil | Cetostearyl alcohol (C16&C18) | 0.5 | 0.5 | 0.5 | 0.5 |

(continued)

| Formulation No. | | Formulation 51 | Formulation 52 | Formulation 53 | Formulation 54 |
|---|---|---|---|---|---|
| Liquid oil ($\alpha$ value) | Liquid paraffin (0°) | 3 | | | |
| | Light liquid paraffin (0°) | | 3 | | |
| | Octyldodecyl myristate (5°) | | | 3 | |
| | Cetyl 2-ethylhexanoate (7°) | | | | 3 |
| Surfactant | | 0.3 | 0.3 | 0.3 | 0.3 |
| Water | Purified water | q.s. | q.s. | q.s. | q. s. |
| Moisturizing ability | Water evaporation suppression rate (%) | 32.91 | 26.31 | 26.74 | 27.15 |
| | Evaluation | Good | Good | Good | Good |
| * Additives (thickener, stabilizer, pH adjuster, and the like) are appropriately added (the total amount of additives is 3 wt.% or less). | | | | | |

As shown in Table 8, when a liquid oil having an $\alpha$ value of 0°, 5°, or 7° was used, the desired moisturizing ability could also be achieved.

**Claims**

1. An emulsion topical composition for skin, comprising:

   (A) benzoyl peroxide;
   (B) 0.3 to 12.5 wt.% of at least one liquid oil having an $\alpha$ value of 0° to 8°;
   (C) 0.3 to 2.5 wt.% of at least one solid oil;
   (D) at least one aqueous solvent; and
   (E) at least one surfactant.

2. The emulsion topical composition according to claim 1, comprising:
   only benzoyl peroxide as an active ingredient.

3. The emulsion topical composition according to claim 1 or 2, wherein the liquid oil (B) is selected from the group consisting of squalane, liquid paraffin, light liquid paraffin, octyldodecyl myristate, decyl oleate, and cetyl 2-ethylhexanoate.

4. The emulsion topical composition according to any one of claims 1 to 3, wherein the solid oil (C) is selected from the group consisting of aliphatic alcohols having 16 or more carbon atoms, stearic acid, paraffin, and white beeswax.

5. The emulsion topical composition according to any one of claims 1 to 4, wherein a content of the liquid oil (B) is 0.5 to 10 wt.%, and a content of the solid oil (C) is 0.3 to 1.4 wt.%.

6. The emulsion topical composition according to any one of claims 1 to 5, wherein the aqueous solvent (D) consists of (d1) 5 to 30 wt.% of polyhydric alcohol and (d2) 50 wt.% or more of water.

7. The emulsion topical composition according to claim 1, comprising:

   0.3 to 12.5 wt.% of squalane as the liquid oil (B);
   0.3 to 2.5 wt.% of aliphatic alcohol selected from the group consisting of stearyl alcohol, cetostearyl alcohol, and cetyl alcohol as the solid oil (C); and
(d1) 5 to 30 wt.% of polyhydric alcohol and (d2) 50 wt.% or more of water as the aqueous solvent (D).

8. The emulsion topical composition according to claim 1, comprising:

   1.5 to 5 wt.% of squalane as the liquid oil (B);
   0.3 to 0.9 wt.% of aliphatic alcohol selected from the group consisting of stearyl alcohol, cetostearyl alcohol, and cetyl alcohol as the solid oil (C); and
   (d1) 10 to 25 wt.% of polyhydric alcohol and (d2) 65 wt.% or more of water as the aqueous solvent (D),
   wherein the total content of components other than the components (A) to (E) is 5 wt.% or less.

9. The emulsion topical composition according to claim 7 or 8, comprising:
   only benzoyl peroxide as an active ingredient.

FIG.1

# EP 4 257 127 A1

| INTERNATIONAL SEARCH REPORT | International application No. |
|---|---|
| | **PCT/JP2021/044378** |

**A. CLASSIFICATION OF SUBJECT MATTER**

*A61K 31/327*(2006.01)i; *A61K 9/10*(2006.01)i; *A61K 47/06*(2006.01)i; *A61K 47/10*(2006.01)i; *A61K 47/12*(2006.01)i; *A61K 47/14*(2006.01)i; *A61K 47/44*(2017.01)i; *A61P 17/00*(2006.01)i; *A61P 17/10*(2006.01)i
FI:  A61K31/327; A61K9/10; A61P17/00; A61P17/10; A61K47/06; A61K47/14; A61K47/10; A61K47/12; A61K47/44

According to International Patent Classification (IPC) or to both national classification and IPC

**B. FIELDS SEARCHED**

Minimum documentation searched (classification system followed by classification symbols)

A61P17/00; A61P17/10; A61K9/10; A61K47/06; A61K47/10; A61K47/12; A61K47/14; A61K47/44; A61K31/327

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 1-268632 A (L'OREAL SA) 26 October 1989 (1989-10-26)<br>claims, page 4, upper left column, second paragraph, examples | 1-9 |
| Y | JP 2007-9199 A (MARUHO CO., LTD.) 18 January 2007 (2007-01-18)<br>claims, paragraphs [0028], [0044], examples | 1-9 |
| Y | JP 2010-513427 A (GALDERMA RESEARCH & DEVELOPMENT) 30 April 2010 (2010-04-30)<br>claims, examples | 1-9 |
| A | US 2006/0135822 A1 (SCHWARZ, J. et al.) 22 June 2006 (2006-06-22)<br>entire text | 1-9 |
| A | WO 2017/068673 A1 (MARUHO CO., LTD.) 27 April 2017 (2017-04-27)<br>entire text | 1-9 |

☐ Further documents are listed in the continuation of Box C.  ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br><br>**21 December 2021** | Date of mailing of the international search report<br><br>**25 January 2022** |
| Name and mailing address of the ISA/JP<br><br>**Japan Patent Office (ISA/JP)**<br>**3-4-3 Kasumigaseki, Chiyoda-ku, Tokyo 100-8915**<br>**Japan** | Authorized officer<br><br><br><br>Telephone No. |

Form PCT/ISA/210 (second sheet) (January 2015)

23

## INTERNATIONAL SEARCH REPORT
### Information on patent family members

International application No.

**PCT/JP2021/044378**

| Patent document cited in search report | | | Publication date (day/month/year) | Patent family member(s) | | | Publication date (day/month/year) |
|---|---|---|---|---|---|---|---|
| JP | 1-268632 | A | 26 October 1989 | US | 4960772 | A | |
| | | | | claims, column 3, lines 49-56, examples | | | |
| | | | | EP | 335115 | A2 | |
| JP | 2007-9199 | A | 18 January 2007 | (Family: none) | | | |
| JP | 2010-513427 | A | 30 April 2010 | US | 2009/0318550 | A1 | |
| | | | | claims, examples | | | |
| | | | | WO | 2008/087348 | A2 | |
| | | | | EP | 2125117 | A2 | |
| | | | | KR | 10-2009-0091305 | A | |
| | | | | CN | 101631594 | A | |
| US | 2006/0135822 | A1 | 22 June 2006 | (Family: none) | | | |
| WO | 2017/068673 | A1 | 27 April 2017 | WO | 2017/069230 | A1 | |
| | | | | entire text | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H0987140 A

- JP 2013241469 A

**Non-patent literature cited in the description**

- *Pharmaceutical Bulletin,* 1954, vol. 2 (2), 163-173

- *Chemistry region,* 1957, vol. 11 (10), 719-725
- *Fragrance journal,* 1981, vol. 50, 79-82